(19) Europäisches Patentamt
European Patent Office
Office européen des brevets

(11) Veröffentlichungsnummer: **0 348 641 B1**

# (12) EUROPÄISCHE PATENTSCHRIFT

(45) Veröffentlichungstag der Patentschrift: **21.06.95**

(51) Int. Cl.$^{6}$: **C07D 475/04**

(21) Anmeldenummer: **89108451.9**

(22) Anmeldetag: **11.05.89**

Die Akte enthält technische Angaben, die nach dem Eingang der Anmeldung eingereicht wurden und die nicht in dieser Patentschrift enthalten sind.

## (54) Verfahren zur Herstellung von Tetrahydrofolaten.

(30) Priorität: **29.06.88 DE 3821875**

(43) Veröffentlichungstag der Anmeldung:
**03.01.90 Patentblatt 90/01**

(45) Bekanntmachung des Hinweises auf die Patenterteilung:
**21.06.95 Patentblatt 95/25**

(84) Benannte Vertragsstaaten:
**AT BE CH DE ES FR GB GR IT LI LU NL SE**

(56) Entgegenhaltungen:
**EP-A- 0 266 042**
**EP-A- 0 293 029**
**US-A- 2 688 018**

**BIOCHEMISTRY, A.L. Lehninger , 2nd ed., Worth Publishers Inc., (1975) Seite 714 ;**

**J.C. Fontecilla-camps et al., JACS, vol 101, (1979), Seite 6114 ;**

**WISSENSCHAFTLICHE TABELLEN, K. Diem und C. Leutner, 7 Aufgabe, Georg Thieme Verlag Stuttgart.**

(73) Patentinhaber: **EPROVA Aktiengesellschaft**
**Im Laternenacker 5**
**CH-8200 Schaffhausen (CH)**

(72) Erfinder: **Müller, Hans Rudolf**
**Beckenwäldli 18**
**CH-8207 Schaffhausen (CH)**
Erfinder: **Ulmann, Martin**
**Steigstrasse 339**
**CH-8447 Dachsen (CH)**
Erfinder: **Conti, Josef**
**Winkelriedstrasse 22**
**CH-8203 Schauffhausen (CH)**
Erfinder: **Mürdel, Günter**
**vor Hegin**
**D-7708 Tengen-Büsslingen (DE)**

EP 0 348 641 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 5,10-Methenyl-(6R)-tetrahydrofolsäure [5,10-CH-(6R)-THF; "(6R)-I"], von biologisch aktiven Tetrahydrofolaten, insbesondere von 5-Formyl- [5-CHO-(6S)-THF] und 5-Methyl-(6S)-tetrahydrofolsäure [5-Me-(6S)-THF] sowie von deren Salzen, insbesondere deren physiologisch gut verträglichen Calcium-, Magnesium-, Natrium- und Kaliumsalzen.

Die genannten (6S)-Tetrahydrofolate [(6S)-5,6,7,8-Tetrahydropteroyl-L-glutaminate] sind die ausreichend stabilen natürlichen biologisch aktiven Formen der Folsäure (Folsäure Cofaktoren).

5-CHO-(6S)-THF ist der Citrovorum-Faktor (= Wachstumsfaktor für Leuconostoc citrovorum).

Im Organismus wird 5-CHO-(6S)-THF über 5,10-Methylen-(6R)-THF in 5-Me-(6S)-THF umgewandelt.

Tetrahydrofolate enthalten 2 asymmetrische Zentren. Bei ihrer Synthese aus Folsäure [N-(Pteroyl)-L-glutaminsäure] liegt das im Glutaminsäur-Rest enthaltene chirale C-Atom in der L-Form vor, während das durch Hydrierung der Doppelbindung in 5,6-Stellung des Pteroyl-Restes entstandene chirale C-Atom in Position 6 [C(6)] in der racemischen, der (6R,S)-Form, vorliegt. Synthetische Tetrahydrofolate bestehen demnach jeweils aus einer 1:1-Mischung von zwei Diastereomeren.

Im natürlichen Vorkommen, z.B. in der Leber, findet man die Tetrahydrofolate nur in der einen diastereomeren Form, wobei die 5-CHO-THF als 5-CHO-(6S)-THF und die 5-Me-THF als 5-Me-(6S)-THF vorliegt.

Die absolute Konfiguration an C (6) der natürlichen Tetrahydrofolsäure ist nach J.C. Fontecilla-Camps et al., J. Amer. chem. Soc. 101 (20), 6114/5 (1979) gemäss der Sequenz-Regel mit S zu spezifizieren und diejenige an C (6) der natürlichen 5,10-Methylen-tetrahydrofolsäure und der 5,10-Methenyl-tetrahydrofolsäure mit R: R. Kalbermatten et al., Helv. chim. Acta 64 (8), 2627 (1981), Fussnote 4.

5-CHO-(6R,S)-THF (Folinsäure), wird in Form ihres Calciumsalzes (Leucovorin) als Arzneimittel verwendet zur Behandlung von megaloblastischer Folsäuremangel-Anämie, als Antidot zur Verstärkung der Verträglichkeit von Folsäure-Antagonisten speziell von Aminopterin, Methotrexat und Fluoruracil in der Krebstherapie ("leucovorin rescue") und der Behandlung von Autoimmunkrankheiten wie Psoriasis und rheumatischer Arthritis sowie zur Verstärkung der Verträglichkeit von bestimmten Antiparasitika, etwa Trimethoprim-Sulfamethoxazol, in der Chemotherapie.

Calcium-5-methyl-(6R,S)-tetrahydrofolat findet eine ähnliche Verwendung.

Nach Verabreichung von 5-CHO-(6R,S)-THF wird der (6S)-Anteil dieser Diastereomeren-Mischung rasch in 5-Me-(6S)-THF umgewandelt, während der (6R)-Anteil nicht metabolisiert wird: J.A. Straw et al., Cancer Research 44, 3114-3119 (1984).

5-CHO-(6R)-THF hemmt zudem einige für den $C_1$-Transfer verantwortliche Enzyme und damit die biochemische Wirkung der Tetrahydrofolate: R.P. Leary et al., Biochem. Biophys. Res. Commun. 56, 484 (1973); V.F. Scott et al., ibid. 14, 523 (1964); G.K. Smith et al., Biochemistry, 20, 4034 (1981). Die Verwendung von (6S)-Tetrahydrofolaten anstelle von (6R,S)-Tetrahydrofolaten müsste daher auch therapeutische Vorteile aufweisen.

Es besteht darum ein Bedürfnis, die bisher verwendeten Mischungen von Diastereomeren, enthaltend jeweils die (6S)- und (6R)-Form, durch die natürliche (65)-Form zu ersetzen.

Es sind mehrfach Anstrengungen zur Trennung von (6R,S)-Tetrahydrofolsäuren und zu deren asymmetrischer Synthese und Isolierung der physiologisch aktiven Formen unternommen worden.

D. Cosulich et al., J. Amer. chem. Soc. 74, 4215-16 (1952), US-PS 2.688.018 (31.08.1954) haben beispielsweise versucht, die Trennung durch fraktionierte Kristallisation eines Erdalkalisalzes, z.B. des Calcium- oder Strontiumsalzes der 5-CHO-(6R,S)-THF aus wässeriger Lösung zu bewerkstelligen [siehe auch J.C. Fontecilla-Camps et al., J. Amer. chem. Soc. 101, 6114 (1979)].

Unter den von D. Cosulich et al. offenbarten Bedingungen lässt sich die gewünschte . Trennung nicht realisieren. Bei der Kristallisation z.B. des Calciumsalzes von 5-CHO-(6R,S)-THF aus Wasser bei pH 7-8 wird immer wieder die 6R,S-Form erhalten, wie sich mittels chromatographischer Analyse an einer chiralen HPLC-Säule sowie anhand der optischen Drehung quantitativ nachweisen lässt. Dabei ist es unerheblich, ob man rohes oder reines Calciumsalz von 5-CHO-(6R,S)-THF zur Kristallisation einsetzt; stets wird die (6R,S)-Form zurückerhalten. Eine Trennung und Anreicherung der (6S)-Form kann auch nicht erreicht werden, wenn man die übersättigte wässerige Lösung eines Erdalkalisalzes von 5-CHO-(6R,S)-THF mit authentischem Erdalkalisalz von 5-CHO-(6S)-THF impft.

Die Trennung der Diastereomeren-Paare wurde auch mittels Chromatographie versucht: J. Feeney et al., Biochemistry 20, 1837 (1981). Ausserdem wurden die (6S)-Isomeren hergestellt durch stereospezifische Reduktion von Dihydrofolaten in Gegenwart von Dihydrofolat-Reduktase: L. Rees et al., Tetrahedron 42 117 (1986).

L. Rees et al., J. Chem. Soc., Chem. Commun. 1987, 470 und EP-A2-0.266.042, haben ein Trennverfahren für (6R,S)-THF beschrieben, mit dessen Hilfe 5-CHO-(6S)-THF und 5-CHO-(6R)-THF in geringen Mengen erzeugt werden konnten. Das Verfahren besteht darin, dass man (6R,S)-THF z.B. mit (-)-Menthylchloroformiat zu den diastereomeren 5-(-)-Menthyloxycarbonyl-tetrahydrofolsäuren umsetzt, diese durch wiederholte Behandlung mit n-Butanol trennt, die erhaltenen Diastereomeren mit einer gesättigten Lösung von Bromwasserstoff in einer Mischung aus Ameisensäure und Essigsäure erwärmt, wobei nach Hydrolyse 5-Formyl-(6S)- und -(6R)-THF gebildet werden, und schliesslich diese als Calciumsalze isoliert.

Dieses Verfahren ist umständlich und schwierig und benötigt zur Herstellung des chiralen Reagens hochgiftiges Phosgen. Zudem ist das Ausgangsmaterial (6R,S)-THF sehr unbeständig. Bei der Abspaltung der chiralen Hilfsgruppe mit HBr in AcOH bei > 50 °C wird ein Teil der Glutaminsäure abgespalten, und es entstehen Nebenprodukte, die sich nur schwierig abtrennen lassen. Die nach einem solchen Verfahren erzeugte (6S)-Folinsäure wäre so teuer, dass deren Anwendung anstelle der (R,S)-Tetrahydrofolate kaum in Betracht kommt.

Bis heute ist kein industriell brauchbares Verfahren zur Gewinnung von (6S)-Tetrahydrofolaten bekannt geworden.
Es bestand somit nach wie vor die Aufgabe, ein einfaches, industriell anwendbares Verfahren zur Herstellung von 5-CHO- und 5-Me-(6S)-THF zu finden.

5-CHO- und 5-Me-(6R,S)-THF können in bekannter Weise aus Folsäure hergestellt werden. Durch Formylierung mit Ameisensäure wird 10-Formyl-folsäure (10-CHO-FA) gebildet. Diese kann anschliessend katalytisch zu 10-Formyl-tetrahydrofolsäure (10-CHO-THF) hydriert werden. Durch Dehydratisierung ist daraus 5,10-Methenyl-tetrahydrofolsäure (I; 5,10-CH-THF; "Anhydro-leucovorin") erhältlich:

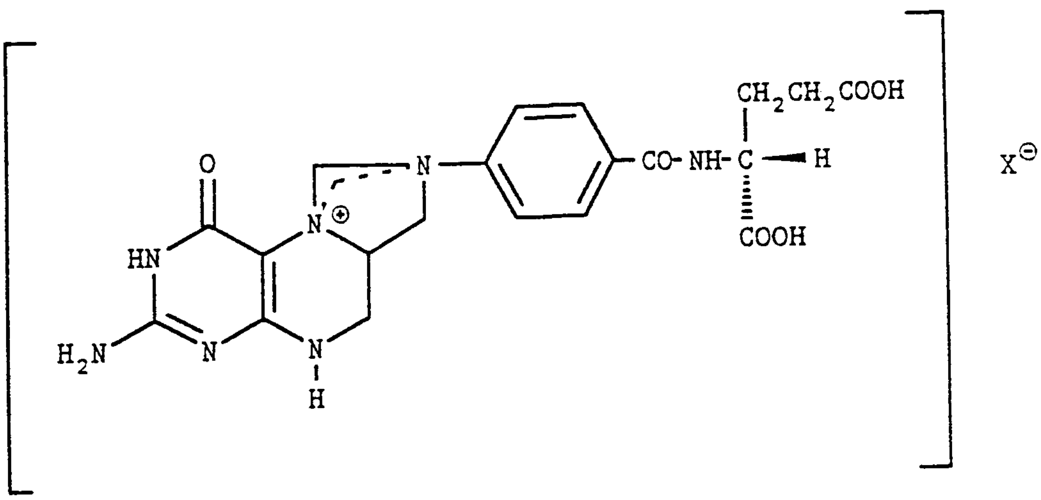
I

In dieser Formel ist $X^{\ominus}$ ein Äquivalent eines beliebigen Anions, z.B. $Cl^{\ominus}$ oder $Br^{\ominus}$. I kann auch in Form eines Säureadditionssalzes, z.B. des Chlorid-Hydrochlorids der Kurzformel $(5,10\text{-CH-THF})^{\oplus}$ $Cl^{\ominus}$•HCl, vorliegen. Aus dieser Form kann mit Hilfe eines Anionenaustauschers auch das entsprechende innere Salz erhalten werden (I, $X^{\ominus}$ fehlt, $COO^{\ominus}$ an Stelle von COOH).

Aus I lassen sich die gewünschten Tetrahydrofolate leicht herstellen: Durch Hydrolyse (Transformylierung) erhält man 5-Formyl-tetrahydrofolsäure (5-CHO-THF), die als Calciumsalz (Leucovorin) isoliert werden kann: CH-PS 305.574 (CYANAMID). Durch Reduktion mit Natriumborhydrid erhält man die 5-Methyltetrahydrofolsäure(5-Me-THF; Mefolinsäure), die als Calcium- oder Magnesiumsalz isoliert werden kann: CH-PS 649.550 (EPROVA).

Es wurde nun überraschend gefunden, dass bei der fraktionierten Kristallisation von Lösungen von (6R,S)-I bzw. von dessen Salzen (6R)-I bzw. dessen Säureadditionssalze (der Kurzformel $[5,10\text{-CH-(6R)-THF}]^{+}X^{-}$•HX) bzw. dessen inneres Salz zur Kristallisation gebracht werden können, derart, dass der Gehalt des Kristallisates an (6R)-I bis zu mehr als 90% beträgt. (6R)-I seinerseits ist zur Herstellung der gewünschten Tetrahydrofolate gut geeignet: man erhält daraus 5-CHO-(6S)-THF durch Hydrolyse und 5-Me-(6S)-THF durch Reduktion.

Entscheidend ist also, dass man die Diastereomerentrennung auf der Stufe von I durchführt.

Das Gelingen dieser Auftrennung ist darum überraschend, weil Rees et al., J. Chem. Soc. Chem. Commun. 1987, 470, zur Ermöglichung der Trennung ein zusätzliches chirales Zentrum in das THF-Molekül

eingebaut und damit eine erhebliche Komplikation in Kauf genommen haben.

Gegenstand der Erfindung ist somit ein Verfahren zur Herstellung von 5,10-Methenyl-(6R)-, 5-Formyl-(6S)- und/oder 5-Methyl-(6S)-tetrahydrofolsäure sowie von deren Salzen, dadurch gekennzeichnet, dass man 5,10-Methenyl-(6R,S)-tetrahydrofolsäure, dessen inneres Salz und/oder eines ihrer Salze mit starken Säuren einer Diastereomerentrennung durch fraktionierte Kristallisation unterzieht und, falls erwünscht, die erhaltene 5,10-Methenyl-(6R)-tetrahydrofolsäure durch Behandeln mit einem hydrolysierenden Mittel in 5-Formyl-(6S)-tetrahydrofolsäure oder durch Behandeln mit einem reduzierenden Mittel in 5-Methyl-(6S)-tetrahydrofolsäure umwandelt und/oder eine erhaltene freie Säure durch Behandeln mit einer Base in eines ihrer Salze überführt.

Die erfindungsgemässe Diastereomeren-Trennung erfolgt vorzugsweise durch fraktionierte Kristallisation aus mindestens einem polaren Lösungsmittel.

Als polare Lösungsmittel kommen beispielsweise in Betracht: niedere aliphatische Carbonsäuren, insbesondere Ameisensäure sowie Essigsäure, flüssige wasserlösliche Amide wie Formamid, Dimethylformamid, Dimethylacetamid, 1-Methyl-pyrrolidon 2-Piperidinon, 4-Formyl-morpholin, 1-Formyl-pyrrolidon, 1-Formyl-piperidin, Tetramethylharnstoff, Sulfoxide oder Sulfone wie Dimethylsulfoxid, Dimethylsulfon oder Sulfolan (Tetramethylensulfon), konzentrierte wässerige starke Säuren wie 20-35%-ige Salzsäure oder andere wässerige Mineralsäuren, wie beispielsweise Bromwasserstoffsäure, Jodwasserstoffsäure, Schwefelsäure, Salpetersäure, Phosphorsäure, oder Lösungen starker organischer Säuren wie von Oxalsäure, Maleinsäure, Trichloressigsäure, Trifluoressigsäure, Methansulfonsäure, Benzolsulfonsäure, Toluolsulfonsäure, Camphersulfonsäuren. Ameisensäure wird bevorzugt.

Bevorzugt eignen sich jedoch Gemische der vorstehend angegebenen Lösungsmittel, insbesondere Gemische eines der angegebenen organischen "Lösungsmittel" mit einer wässerigen Mineralsäure oder mit Wasser ("Kristallisationsmittel").

Als Lösungsmittel kann auch eine konzentrierte wässerige Mineralsäure, beispielsweise konzentrierte Salzsäure und als Kristallisationsmittel eine geringe Menge Wasser verwendet werden. Wenn man dabei die Wassermenge derjenigen der konzentrierten wässerigen Mineralsäure annähert oder gar gleichsetzt, so kristallisiert [5,10-(6R,S)-THF]$^+$X$^-$•HX aus, d.h. eine Trennung erfolgt nicht. Bevorzugtes Lösungsmittel ist Ameisensäure und bevorzugtes Kristallisationsmittel eine wässerige starke Säure, insbesondere verdünnte Salzsäure, Bromwasserstoffsäure, Schwefelsäure, eine Sulfonsäure oder Salpetersäure. Anstelle einer wässerigen starken Säure kann als Kristallisationsmittel auch Eisessig verwendet werden.

Als Endprodukt erhält man in der Regel ein Säureadditionssalz des 5,10-Methenyl-(6R)-tetrahydrofolsäuresalzes I mit der entsprechenden Säure HX. Versetzt man z.B. eine konzentrierte Lösung des Hydrochlorids von (6R,S)-I (X = Cl) in Ameisensäure ("Lösungsmittel") mit wässeriger konzentrierter Salzsäure ("Kristallisationsmittel"), so kristallisiert (6R)-I in Form des Chlorid-Hydrochlorids der Kurzformel [5,10-CH-(6R)-THF]Cl•HCl mit einem (6R)-Anteil von etwa 90% aus. Dieser (6R)-Anteil kann durch nochmalige Umkristallisation, z.B. aus Ameisensäure/Salzsäure, gesteigert werden.

Analog erhält man z.B. das Hydrobromid von (6R)-I (X = Br), wenn man eine Lösung des Hydrobromids von (6R,S)-I (X = Br) oder des inneren Salzes von (6R,S)-I in Ameisensäure mit wässeriger Bromwasserstoffsäure versetzt.

Säurekonzentration und Mengenverhältnisse von Lösungsmittel und Kristallisationsmittel können innerhalb weiter Grenzen variiert werden. Vorzugsweise liegt die Konzentration von 5,10-CH-(6R,S)-THF zwischen 10 und 40 Gew.%, das Verhältnis zwischen Lösungs- und Kristallisationsmittel zwischen 10:1 und 1:1. Die Säurekonzentration im Kristallisationsmittel kann zwischen 0 und 12 N liegen. In der Regel kann man höhere Ausbeuten bei geringerem Trennungseffekt erzielen, wenn man relativ viel Kristallisationsmittel verwendet; wird dagegen wenig Kristallisationsmittel eingesetzt, so ist der Trenneffekt besser, wobei allerdings die Ausbeute sinken kann. Abhängig von dem angestrebten Ziel, dem jeweiligen Ausgangsmaterial, Lösungs- und Kristallisationsmittel kann man das Optimum der Reaktionsbedingungen durch systematische Versuche ohne Schwierigkeiten bestimmen.

Geht man von einem Säureadditionssalz des Typs [5,10-CH-(6R,S)-THF]X•HX aus, so kann die Diastereomerentrennung unter Bildung von [5,10-CH-(6R)-THF]X•HX auch bereits gelingen durch fraktionierte Kristallisation aus einem Lösungsmittel wie Ameisensäure unter Zusatz von Wasser oder auch ohne jeden Zusatz eines Kristallisationsmittels.

In der Regel kristallisiert die gewünschte (6R)-Verbindung zuerst aus; die diastereomere (6S)-Verbindung reichert sich in den Filtraten an. In einzelnen Fällen, z.B. wenn man das innere Salz von 5,10-CH-(6R,S)-THF aus Ameisensäure/Wasser oder wenn man das [5,10-CH-(6R,S)-THF]•Trichloracetat aus Dimethylsulfoxid kristallisiert, fällt dagegen zunächst das (6S)-Diastereomere aus, während man das (6R)-Diastereomere aus dem Filtrat isolieren kann.

Eine besonders bevorzugte Ausführungsform der Erfindung besteht darin, dass man eine durch Formylierung von Folsäure zu 10-Formyl-folsäure und anschliessende katalytische Hydrierung und Dehydratisierung erhaltene Ameisensäure-Lösung, die (6R,S)-I in situ enthält, mit einer starken wässerigen Säure, z.B. Salzsäure, versetzt; dadurch kann das (6R)-Diastereomere in Form des Säureadditionssalzes der Formel [5,10-CH-(6R)-THF]X•HX, z.B. das entsprechende Chlorid-Hydrochlorid, zur Kristallisation gebracht werden.

Durch dieses Verfahren sind die (6S)-Tetrahydrofolate, insbesondere 5-CHO-(6S)-THF und 5-Me-(6S)-THF, sehr einfach und besonders wirtschaftlich zugänglich geworden.

Als physiologisch gut verträgliche Salze kommen in erster Linie in Betracht die Calcium-, Magnesium-, Natrium- und Kaliumsalze.

Die Magnesiumsalze zeichnen sich durch ihre verglichen mit den Calciumsalzen im allgemeinen erhöhten Wasserlöslichkeiten aus. Dies erlaubt bei ihrer Anwendung einen grösseren Dosisspielraum.

Es ist nicht erforderlich, 5,10-CH-(6R)-THF oder deren Säureadditionssalze vollständig von begleitender (6S)-Form zu befreien. Bei der Überführung durch Hydrolyse oder Reduktion in 5-CHO-(6S)-THF bzw. 5-Me-(6S)-THF und deren Isolierung wird jeweils die Restmenge an "falschem" Diastereomerem eliminiert.

Beispiel 1 **Calcium-5-Formyl-(6S)-tetrahydrofolat**

**1.1 Fraktionierte Kristallisation von 5,10-Methenyl-(6R,S)-tetrahydrofolsäure [Anhydro-leucovorin; (6R,S)-I)]**

Eine Lösung von 1000 g [5,10-Methenyl-(6R,S)-tetrahydrofolsäure]chlorid•hydrochlorid•dihydrat ([5,10-CH-(6R,S)-THF]Cl• HCl•2 $H_2O$) in 2 lt Ameisensäure von 35 °C wird mit 300 ml 2 N Salzsäure yersetzt, langsam auf 20 °C abgekühlt und kristallisieren gelassen. Nach einigen Stunden wird das ausgeschiedene Produkt abfiltriert und mit 2 N Salzsäure gewaschen. Man erhält (6R)-I mit einem Diastereomeren-Gehalt von 91,5% an [5,10-CH-(6R)-THF]Cl•HCl, bestimmt mittels HPLC unter Verwendung einer chiralen Säule (Resolvosil-BSA-7).

$[\alpha]_D^{25}$ = +36 ° (c = 1 in DMSO/2 N HCl 8:2)

**1.2 Überführung in Calcium-5-formyl-(6S)-tetrahydrofolat (Transformylierung)**

120 g des nach 1.1 erhaltenen Kristallisates werden in wässeriger Natronlauge gelöst, einige Stunden bei pH 5,5 bis 6,5 schwach gekocht, abgekühlt und mit überschüssigem Calciumchlorid versetzt. Das ausgeschiedene Calciumsalz von 5-CHO-(6S)-THF wird aus Wasser umkristallisiert. Man erhält Calcium-5-Formyl-(6S)-tetrahydrofolat•5 $H_2O$. Gehalt an Ca-5-CHO-(6S)-THF ≧ 99 Flächen%, bestimmt mittels HPLC unter Verwendung einer chiralen Säule.

$[\alpha]_D^{22}$ = -15 ° (bezogen auf wasserfreies Ca-Salz; c = 1,5% in Wasser).

Beispiel 2 **5-Methyl-(6S)-tetrahydrofolsäure und Calcium 5-Methyl-(6S)-tetrahydrofolat**

**2.1 Fraktionierte Kristallisation von in situ erhaltenem (6R,S)-I**

Eine Lösung, die durch katalytische Hydrierung von 10-Formyl-folsäure in Ameisensäure erhalten wurde, wird durch Eindampfen auf einen Gehalt von etwa 30-35 % konzentriert. Ein Gewichtsteil des Konzentrates wird bei 35 °C mit etwa 0,4 Gewichtsteilen konzentrierter (35%iger) Salzsäure versetzt. Man lässt auf Raumtemperatur abkühlen, lässt über Nacht bei 5-10 ° stehen, filtriert das ausgeschiedene Kristallisat ab und wäscht es mit wenig eiskalter Salzsäure und Aceton. Man erhält [5,10-CH-(6R)-THF]Cl•HCl; (6R)-Anteil. 90%.

**2.2 Rekristallisation von rohem [5,10-CH-(6R)-THF]Cl•HCl**

Man löst 1,2 kg des nach 2.1 erhaltenen Kristallisats bei 35 ° C in 3 lt Ameisensäure, versetzt mit 450 ml 2 N Salzsäure, kühlt allmählich auf Raumtemperatur ab und hält einige Stunden bei 0-5 °C. Das gebildete Kristallisat wird abfiltriert und mit verdünnter Salzsäure und Aceton gewaschen. Man erhält [5,10-CH-(6R)-THF]Cl•HCl•2 $H_2O$ mit einem (6R)-Anteil von 98 %.

**2.3 5-Methyl-(6S)-tetrahydrofolsäure [5-Me-(6S)-THF]**

Man trägt 30 g des nach 2.2 erhaltenen [5,10-CH-(6R)-THF]Cl• HCl•2 $H_2O$ in eine Lösung von 25 g Natriumborhydrid und 3,4 g Tris in 300 ml Wasser ein und rührt über Nacht. Die erhaltene Lösung wird mit Salzsäure bis zum pH 4 angesäuert, wobei sich 5-Me-(6S)-THF als Dihydrat ausscheidet. Es wird abfiltriert und mit Wasser gewaschen.

**2.4 Calcium-5-methyl-(6S)-tetrahydrofolat**

Die Hälfte des nach 2.3 erhaltenen 5-Me-(6S)-THF•2 $H_2O$ wird in möglichst wenig verdünnter Natronlauge gelöst, die Lösung wird mit Aktivkohle und Filterhilfsmittel behandelt, filtriert und mit überschüssigem Calciumchlorid versetzt. Das gebildete Calcium-5-methyl-(6S)-tetrahydrofolat-pentahydrat scheidet sich

allmählich aus. Nach Kristallisierenlassen bei 0-3°C wird das Produkt abfiltriert und aus Wasser umkristallisiert.
$[\alpha]_D^{25}$ = +40° (bezogen auf die wasserfreie Verbindung; c= 1,5% in Wasser).
Dieselbe Verbindung wird auch erhalten, wenn man das nach Beispiel 1.1 erhaltene [5,10-CH-(6R)-THF]-Cl• HCl nach Beispiel 2.3/2.4 weiter umsetzt.

Beispiel 3 **Magnesium-5-methyl-(6S)-tetrahydrofolat**

Die zweite Hälfte der nach Beispiel 2.3 erhaltenen 5-Me-(6S)-THF wird allmählich in der minimal erforderlichen Menge 2 N Natronlauge gelöst und mit einem Überschuss an Magnesiumchlorid versetzt. Durch Zusatz von Ethanol wird das Magnesiumsalz zur Kristallisation gebracht. Diese neue Verbindung ist gut wasserlöslich.

Beispiel 4 **[5,10-CH-(6R)-THF]Cl• HCl**

**4.1 Fraktionierte Kristallisation von [5,10-CH-(6R,S)-THF]Cl•HCl aus Ameisensäure**
Man löst 25 g [5,10-CH-(6R,S)-THF]Cl•HCl•2 $H_2O$ in 50 ml 35°C warmer Ameisensäure, kühlt auf 0°C ab und impft mit einer Spur von nach Beispiel 2.2 erhaltenem Produkt. Nach einigen Stunden wird das erhaltene [5,10-CH-(6R)-THF]Cl•HCl abfiltriert; (6R)-Anteil 79%. Durch erneutes Umkristallisieren steigt der (6R)-Anteil auf über 90 %.
**4.2 Selektive Kristallisation von [5,10-CH-(6R)-THF]Cl•HCl durch Zusatz von Wasser**
Man löst 25 g [5,10-CH-(6R,S)-THF]Cl•HCl•2 $H_2O$ in 50 ml Ameisensäure bei 35°C, versetzt mit 5 ml Wasser, kühlt auf 0°C ab und impft mit authentischem (6R)-Isomerem. Nach Stehen über Nacht wird das erhaltene [5,10-CH-(6R)-THF]Cl•HCl abfiltriert; (6R)-Anteil: 86,5%.
**4.3 Selektive Kristallisation von [5,10-CH-(6R)-THF]•Cl• HCl aus wässeriger Salzsäure**
Man löst 10 g [5,10-CH-(6R,S)-THF]Cl•HCl•2 $H_2O$ bei 50°C in 90 ml konzentrierter Salzsäure, verdünnt mit 10 ml Wasser und impft gleichzeitig mit authentischem Material. [5,10-CH-(6R)-THF]Cl•HCl•2 $H_2O$ kristallisiert aus; 6R-Anteil: 87%.

Beispiel 5 **Herstellung des innern Salzes der 5,10-CH-(6R,S)-THF und von Salzen der 5,10-CH-(6R,S)-THF mit starken Säuren sowie von [5,10-CH-(6S)-THF]Br• HBr**

**5.1 Inneres Salz der 5,10-CH-(6R,S)-THF**
Eine Lösung von 200 g [5,10-CH-(6R,S)-THF]Cl•HCl in 1000 ml Ameisensäure wird durch eine mit 1100 ml Anionenaustauscherharz (IRA-68) beschickte Säule perkolieren gelassen. Das Effluat wird unter vermindertem Druck eingedampft. Der Rückstand wird in Ameisensäure gelöst und die Lösung in mehrere aliquote Teile aufgetrennt.
Ein Teil der Lösung wird mit Ethanol versetzt, wobei das innere Salz auskristallisiert. Es ist auffallend gelb gefärbt. Es ist in Wasser, organischen Lösungsmitteln und auch in polaren Lösungsmitteln mit Ausnahme von Ameisensäure sehr wenig löslich.
Das innere Salz von 5,10-CH-(6R,S)-THF kann auch erhalten werden, wenn man eine nach Beispiel 2.1 in situ erhaltene konzentrierte Lösung von 5,10-CH-(6R,S)-THF durch Zusatz eines niedrigen Alkohols, z.B. Ethanol, oder eines Ketons, z.B. Methylpropylketon, zur Kristallisation bringt.
**5.2 Salze von 5,10-CH-(6R,S)-THF mit starken Säuren**
Aliquote Teile der nach 5.1 erhaltenen Lösung werden mit einem Überschuss der gewünschten Säure in Ameisensäure versetzt und durch Zusatz von Ethanol gefällt. Die erhaltenen Salze werden abfiltriert.
Nach dieser Methode werden hergestellt:
[5,10-CH-(6R,S)-THF]Br•HBr,
[5,10-CH-(6R,S)-THF]J•HJ,
[5,10-CH-(6R,S)-THF]-Phosphat,
[5,10-CH-(6R,S)-THF]-Sulfat,
[5,10-CH-(6R,S)-THF]-Nitrat,
[5,10-CH-(6R,S)-THF]-Oxalat,
[5,10-CH-(6R,S)-THF]-Maleinat,
[5,10-CH-(6R,S)-THF]-Toluol-4-sulfonat,
]5,10-CH-(6R,S)-THF]-Benzolsulfonat,
[5,10-CH-(6R,S)-THF]-Methansulfonat,
[5,10-CH-(6R,S)-THF]-Trichloracetat und

[5,10-CH-(6R,S)-THF]-Trifluoracetat.

**5.3 [5,10-CH-(6R)-THF]Br•HBr**

Eine Lösung von 20 g innerem Salz der [5,10-CH-(6R,S)-THF] in 30 ml Ameisensäure wird mit etwas mehr als der berechneten Menge 6 N wässeriger Bromwasserstoffsäure versetzt und bei 20-25°C gerührt. Nach einiger Zeit scheidet sich [5,10-Methenyl-(6R)-tetrahydrofolsäure]bromid-hydrobromid aus; (6R)-Anteil: 89%.

[5,10-CH-(6R)-THF]Br•HBr wird auch erhalten, wenn man [5,10-CH-(6R,S)-THF]Br•HBr in Ameisensäure löst und 2 N wässerige Bromwasserstoffsäure zusetzt oder wenn man das "racemische" Bromid-hydrobromid aus wenig Dimethylsulfoxid (etwa 4 ml pro g) fraktioniert kristallisiert.

Bei den Säureadditionssalzen mit Halogenwasserstoffsäuren, Salpetersäure, Schwefel- und Sulfonsäuren können zwei Säureäquivalente pro Mol 5,10-CH-THF an der Salzbildung beteiligt sein. Bei den Salzen mit mittelstarken Säuren, z.B. Maleinsäure, ist jeweils nur ein Säureäquivalent in Salzform gebunden.

<u>Beispiel 6</u> **[5,10-CH-(6R)-THF]J•HJ**

Diese Verbindung wird analog Beispiel 5 hergestellt.

<u>Beispiel 7</u> **[5,10-CH-(6R)-THF]-Phosphat**

Man löst 30 g [5,10-CH-(6R,S)-THF]-Phosphat, hergestellt nach Beispiel 5, bei 50°C in 80 ml Dimethylsulfoxid und rührt einige Stunden bei Raumtemperatur. Nach längerer Zeit kristallisiert [5,10-CH-(6R)-THF]-Phosphat aus, welches abfiltriert und getrocknet wird.

6R-Isomerenanteil: 70% (HPLC).

[5,10-CH-(6R)-THF]-Phosphat wird auch erhalten, wenn man eine Lösung des racemischen Phosphats in Ameisensäure mit einer geringen Menge von 2 M wässeriger Phosphorsäure versetzt.

<u>Beispiel 8</u> **[5,10-CH-(6R)-THF]-Maleinat**

Man löst 30 g [5,10-CH-(6R,S)-THF]-Maleinat bei 50°C in 125 ml 1-Methyl-2-pyrrolidon und rührt anschliessend bei Raumtemperatur. Das nach langer Zeit ausgeschiedene [5,10-CH-(6R)-THF]-Maleinat wird abfiltriert; R-Anteil: 78%.

<u>Beispiel 9</u> **[5,10-CH-(6R)-THF]-Toluol-4-sulfonat**

Man löst 15 g [5,10-CH-(6R,S)-THF]-Toluol-4-sulfonat bei 40-50°C in 28 ml Ameisensäure, versetzt mit 100 ml Wasser und rührt anschliessend bei Raumtemperatur. Das ausgeschiedene [5,10-CH-(6R)-THF]-Toluol-4-sulfonat wird abfiltriert; 6R-Anteil: 85% (HPLC).

[5,10-CH-(6R)-THF]-Toluol-4-sulfonat wird auch erhalten, wenn man die R,S-Verbindung aus 1-Methyl-2-pyrrolidon (etwa 4-6 ml/g) oder Dimethylacetamid oder Dimethylsulfoxid (jeweils etwa 2-3 ml/g) umkristallisiert.

<u>Beispiel 10</u> **[5,10-CH-(6R)-THF]-Trichloracetat**

Eine Lösung von 10 g [5,10-CH-(6R,S)-THF]-Trichloracetat in 50 ml Dimethylsulfoxid wird während einiger Stunden gerührt und anschliessend filtriert. Der Filterrückstand besteht stark überwiegend aus [5,10-CH-(6S)-THF]-Trichloracetat. Das Filtrat wird mit Wasser oder Ethanol versetzt, worauf sich [5,10-CH-(6R)-THF]-Trichloracetat ausscheidet; 6R-Anteil: 70% (HPLC).

<u>Beispiel 11</u> **Inneres Salz der 5,10-CH-(6S)-THF und der 5,10-CH-(6R)-THF**

Eine Lösung von 10 g innerem Salz der 5,10-CH-(6R,S)-THF in 20 ml Ameisensäure wird mit 50 ml Wasser versetzt und 17 Stunden bei 20°C gerührt.

Das ausgeschiedene Kristallisat wird abfiltriert. Es besteht vorwiegend aus dem inneren Salz der 5,10-CH-(6S)-THF. Gehalt an 6S-Form: 73%. Durch Wiederholung der Operation wird der Gehalt an 6S-Form auf 90% gesteigert. Die Mutterlauge wird mit Aceton versetzt, worauf sich ein zweites Kristallisat ausscheidet. Es wird abfiltriert und untersucht. Es besteht zu 75% aus dem inneren Salz von 5,10-CH-(6R)-THF.

Beispiel 12 **[5,10-CH-(6R)-THF]Cl•HCl**

Eine Lösung von 100 g innerem Salz der 5,10-CH-(6R,S)-THF in 200 ml Ameisensäure wird mit 500 ml Wasser versetzt und 20 Stunden gerührt. Das ausgeschiedene innere Salz der 5,10-CH-(6S)-THF wird abfiltriert. Das Filtrat wird mit starker Salzsäure versetzt, oder HCl-Gas wird in das Filtrat eingeleitet. Die allmählich entstehende Ausscheidung besteht aus [5,10-CH-(6R)-THF]Cl•HCl•2 $H_2O$ mit einem Gehalt an 6R-Form von 95%.

Beispiele 13-18

In ähnlicher Weise wie in den Beispielen 5 bis 12 beschrieben lassen sich herstellen:

13. [5,10-CH-(6R)-THF]-Nitrat,
14. [5,10-CH-(6R)-THF]-Sulfat,
15. [5,10-CH-(6R)-THF]-Oxalat,
16. [5,10-CH-(6R)-THF]-Methansulfonat,
17. [5,10-CH-(6R)-THF]-Benzolsulfonat,
18. [5,10-CH-(6R)-THF]-Trifluoracetat.

**Patentansprüche**

**1.** Verfahren zur Herstellung von 5,10-Methenyl-(6R)-, 5-Formyl-(6S)- oder 5-Methyl-(6S)-tetrahydrofolsäure oder von deren Salzen, dadurch gekennzeichnet, dass man 5,10-Methenyl-(6R,S)-tetrahydrofolsäure oder eines ihrer Salze mit starken Sauren fraktioniert kristallisiert und, falls erwünscht, die erhaltene 5,10-Methenyl-(6R)-tetrahydrofolsäure durch Behandeln mit einem hydrolysierenden Mittel in 5-Formyl-(6S)-tetrahydrofolsäure oder durch Behandeln mit einem Alkaliborhydrid oder komplexen Borhydridderivat zu 5-Methyl-(6S)-tetrahydrofolsäure umwandelt oder eine erhaltene freie Säure durch Behandeln mit einer Base in eines ihrer Salze überführt.

**2.** Verfahren zur Herstellung von 5,10-Methenyl-(6R)-, 5-Formyl-(6S)- oder 5-Methyl-(6S)-tetrahydrofolsäure oder von deren Salzen, dadurch gekennzeichnet, dass man das innere Salz von 5,10-Methenyl-(6R,S)-tetrahydrofolsäure fraktioniert kristallisiert, die erhaltene 5,10-Methenyl-(6S)-tetrahydrofolsäure abtrennt und aus der Mutterlauge 5,10-Methenyl-(6R)-tetrahydrofolsäure isoliert und, falls erwünscht, durch Behandeln mit einem hydrolysierenden Mittel in 5-Formyl-(6S)-tetrahydrofolsäure oder durch Behandeln mit einem Alkaliborhydrid oder komplexen Borhydrid-derivat zu 5-Methyl-(6S)-tetrahydrofolsäure umwandelt oder eine erhaltene freie Säure durch Behandeln mit einer Base in eines ihrer Salze überführt.

**3.** Verfahren nach mindestens einem der Ansprüche 1 oder 2, dadurch gekennzeichnet, dass man die Diastereomerentrennung durch fraktionierte Kristallisation in einem polaren Lösungsmittel oder einem Gemisch von polaren Lösungsmitteln durchführt.

**4.** Verfahren nach mindestens einem der Ansprüche 1 oder 2, dadurch gekennzeichnet, dass man die Diastereomeren aus einem polaren Lösungsmittel oder einem Gemisch aus polaren Lösungsmitteln durch den Zusatz eines Kristallisationsmittels fraktioniert kristallisiert.

**5.** Verfahren nach mindestens einem der Ansprüche 1, 2, 3 oder 4, dadurch gekennzeichnet, dass man für die fraktionierte Kristallisation als polares Lösungsmittel ein polares organisches Lösungsmittel und als Kristallisationsmittel eine wässerige Mineralsäure oder Wasser oder als Lösungsmittel eine konzentrierte wässerige Mineralsäure und als Kristallisationsmittel Wasser verwendet.

**6.** Verfahren nach mindestens einem der Ansprüche 1, 2, 3, 4 oder 5, dadurch gekennzeichnet, dass man als Lösungsmittel Ameisensäure und als Kristallisationsmittel eine wässerige starke Säure verwendet.

**7.** Verwendung von nach mindestens einem der Ansprüche 1, 2, 3, 4, 5 oder 6 hergestellter 5,10-Methenyl-(6R)-tetrahydrofolsäure zur Herstellung von 5-Formyl-(6S)-tetrahydrofolsäure durch Hydrolyse.

8. Verwendung von nach mindestens einem der Ansprüche 1, 2, 3, 4, 5 oder 6 hergestellter 5,10-Methenyl-(6R)-tetrahydrofolsäure zur Herstellung von 5-Methyl-(6S)-tetrahydrofolsäure durch Reduktion.

9. Inneres Salz der 5,10-Methenyl-(6R)-tetrahydrofolsäure sowie [5,10-CH-(6R)-THF]-Phosphat, -Sulfat, -Toluolsulfonat, -Oxalat, -Maleinat und -Trichloracetat.

## Claims

1. Process for the preparation of 5,10-methenyl-(6R)-, 5-formyl-(6S)- or 5-methyl-(6S)-tetrahydrofolic acid or of the salts thereof, characterized in that 5,10-methenyl-(6R,S)-tetrahydrofolic acid or one of the salts thereof with strong acids, is fractionally crystallized and, if desired, the resulting 5,10-methenyl-(6R)-tetrahydrofolic acid is converted by treatment with a hydrolysing agent into 5-formyl- (6S) -tetrahydrofolic acid or by treatment with an alkali metal borohydride or complex borohydride derivative to 5-methyl-(6S)-tetrahydrofolic acid, or a resulting free acid is converted into one of its salts by treatment with a base.

2. Process for the preparation of 5,10-methenyl-(6R)-, 5-formyl-(6S)- or 5-methyl-(6S)-tetrahydrofolic acid or of the salts thereof, characterized in that the inner salt of 5,10-methenyl-(6R,S)-tetrahydrofolic acid is fractionally crystallized, the resulting 5,10-methenyl-(6S)-tetrahydrofolic acid is separated off and 5,10-methenyl-(6R)-tetrahydrofolic acid is isolated from the mother liquor and, if desired, is converted by treatment with a hydrolysing agent into 5-formyl-(6S)-tetrahydrofolic acid or by treatment with an alkali metal borohydride or complex borohydride derivative to 5-methyl-(6S)-tetrahydrofolic acid, or a resulting free acid is converted into one of its salts by treatment with a base.

3. Process according to at least one of Claims 1 or 2, characterized in that the diastereomer separation is carried out by fractional crystallization in a polar solvent or a mixture of polar solvents.

4. Process according to at least one of Claims 1 or 2, characterized in that the diastereomers are fractionally crystallized from a polar solvent or a mixture of polar solvents by the addition of a crystallizing agent.

5. Process according to at least one of Claims 1, 2, 3 or 4, characterized in that used for the fractional crystallization is a polar organic solvent as polar solvent, and an aqueous mineral acid or water as crystallizing agent, or a concentrated aqueous mineral acid as solvent, and water as crystallizing agent.

6. Process according to at least one of Claims 1, 2, 3, 4 or 5, characterized in that formic acid is used as solvent, and an aqueous strong acid is used as crystallizing agent.

7. Use of 5,10-methenyl-(6R)-tetrahydrofolic acid prepared according to at least one of Claims 1, 2, 3, 4, 5 or 6 for the preparation of 5-formyl-(6S)-tetrahydrofolic acid by hydrolysis.

8. Use of 5,10-methenyl-(6R)-tetrahydrofolic acid prepared according to at least one of Claims 1, 2, 3, 4, 5 or 6 for the preparation of 5-methyl-(6S)-tetrahydrofolic acid by reduction.

9. Inner salt of 5,10-methenyl-(6R)-tetrahydrofolic acid, and [5,10-CH-(6R)-THF] phosphate, sulphate, toluenesulphonate, oxalate, maleate and trichloroacetate.

## Revendications

1. Procédé de préparation d'acides 5,10-méthényl-(6R)-, 5-formyl-(6S)- ou 5-méthyl-(6S)-tétrahydrofoliques ou de leurs sels, caractérisé en ce qu'on cristallise de façon fractionnée l'acide 5,10-méthényl-(6R,S)-tétrahydrofolique ou un de ses sels avec des acides forts et, si c'est souhaité, on transforme l'acide 5,10-méthényl-(6R)-tétrahydrofolique par traitement avec un agent hydrolysant en acide 5-formyl-(6S)-tétrahydrofolique ou par traitement avec un borohydrure alcalin ou un dérivé borohydrure complexe on transforme en acide 5-méthyl-(6S)-tétrahydrofolique ou on transforme un acide libre obtenu par traitement avec une base en un de ses sels.

2. Procédé de préparation d'acides 5,10-méthényl-(6R)-, 5-formyl-(6S)- ou 5-méthyl(6S)-tétrahydrofoliques ou de leurs sels, caractérisé en ce qu'on fait cristalliser de façon fractionnée le sel interne de l'acide 5,10-méthényl-(6R,S)-tétrahydrofolique, on sépare l'acide 5,10-méthényl-(6S)-tétrahydrofolique obtenu et on isole de la solution mère l'acide 5,10-méthényl-(6R)-tétrahydrofolique et, si c'est souhaité, on transforme par traitement avec un agent hydrolysant en acide 5-formyl-(6S)-tétrahydrofolique ou par traitement avec un borohydrure alcalin ou un dérivé complexe de borohydrure on transforme en acide 5-méthyl-(6S)-tétrahydrofolique ou on transforme un acide libre obtenu, par traitement avec une base en un de ses sels.

3. Procédé selon au moins l'une des revendications 1 ou 2, caractérisé en ce qu'on réalise le fractionnement des diastéréoisomères par cristallisation fractionnée dans un solvant polaire ou dans un mélange de solvants polaires.

4. Procédé selon au moins l'une des revendications 1 ou 2, caractérisé en ce qu'on cristallise les diastéréoisomères dans un solvant polaire ou un mélange de solvants polaires par l'addition d'un agent de cristallisation, de façon fractionnée.

5. Procédé selon au moins l'une des revendications 1, 2, 3 ou 4, caractérisé en ce qu'on utilise pour la cristallisation fractionnée comme solvant polaire un solvant organique polaire et comme agent de cristallisation une solution aqueuse d'acide minéral ou de l'eau ou comme solvant une solution aqueuse concentrée d'acide minéral et comme agent de cristallisation de l'eau.

6. Procédé selon au moins l'une des revendications 1, 2, 3, 4 ou 5, caractérisé en ce qu'on utilise comme solvant de l'acide formique et comme agent de cristallisation une solution aqueuse d'acide fort.

7. Utilisation d'acide 5,10-méthényl-(6R)-tétrahydrofolique préparé selon au moins l'une des revendications 1, 2, 3, 4, 5 ou 6 pour préparer de l'acide 5-formyl-(6S)-tétrahydrofolique par hydrolyse.

8. Utilisation d'acide 5,10-méthényl-(6R)-tétrahydrofolique préparé selon au moins l'une des revendications 1,2,3, 4, 5 ou 6 pour préparer de l'acide 5-méthyl-(6S)-tétrahydrofolique par réduction.

9. Sel interne de l'acide 5,10-méthényl-(6R)-tétrahydrofolique ainsi que [5,10-CH-(6R)-THF]-phosphate, -sulfate, -toluènesulfonate, -oxalate, - maléinate et -trichloroacétate.